(19) Europäisches Patentamt
European Patent Office
Office européen des brevets

(11) Veröffentlichungsnummer: **0 318 849**
**A2**

(12) # EUROPÄISCHE PATENTANMELDUNG

(21) Anmeldenummer: **88119640.6**

(51) Int. Cl.4: **C07C 103/30 , A61K 31/16**

(22) Anmeldetag: **25.11.88**

Patentansprüche für folgenden Vertragsstaat: ES + GR.

(30) Priorität: **04.12.87 DE 3741056**

(43) Veröffentlichungstag der Anmeldung: **07.06.89 Patentblatt 89/23**

(84) Benannte Vertragsstaaten: **AT BE CH DE ES FR GB GR IT LI LU NL SE**


(71) Anmelder: **BEHRINGWERKE Aktiengesellschaft**
**Postfach 1140**
**D-3550 Marburg 1(DE)**

(72) Erfinder: **Zeeck, Axel, Prof. Dr.**
**Brüder-Grimm-Allee 22**
**D-3400 Göttingen(DE)**
Erfinder: **Thiericke, Ralf**
**Leibnizstrasse 5**
**D-3400 Göttingen(DE)**
Erfinder: **Zähner, Hans, Prof. Dr.**
**Im Hopfengarten 1**
**D-7400 Tübingen(DE)**
Erfinder: **Dickneite, Gerhard, Dr.**
**Zum Neuen Hieb 31**
**D-3550 Marburg(DE)**
Erfinder: **Sedlacek, Hans Harald, Dr.**
**Sonnenhang 3**
**D-3550 Marburg(DE)**

(74) Vertreter: **Meyer-Dulheuer, Karl-Hermann, Dr. et al**
**HOECHST Aktiengesellschaft Zentrale Patentabteilung Postfach 80 03 20**
**D-6230 Frankfurt/Main 80(DE)**


(54) **Manumycin-Derivate, Verfahren zu ihrer Herstellung und ihre Verwendung.**

EP 0 318 849 A2

(57)

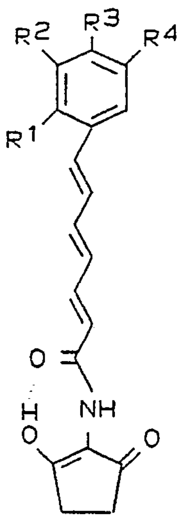

I

wobei
$R^1$ = H oder OH;
$R^2$ = H oder OH;
$R^3$ = H, OH, $OCH_3$, $CH_3$ oder $NH_2$;
$R^4$ = H, OH, $OCH_3$, $NH_2$ oder

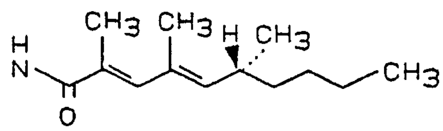

Die Verbindungen der Formel I werden durch Fermentation in Gegenwart substituierter Benzoesäuren der allgemeinen Formel II

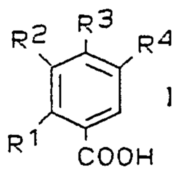

II

wobei
$R^1$ = H oder OH;
$R^2$ = H oder OH;
$R^3$ = H, OH, $OCH_3$, $CH_3$ oder $NH_2$;
$R^4$ = H, OH, $OCH_3$ oder $NH_2$ ist,

durch den Stamm Streptomyces parvulus DSM 40722 hergestellt, hemmen die Leukozyten-Elastase und sind als Arzneimittel verwendbar.

## Manumycin-Derivate, Verfahren zu ihrer Herstellung und ihre Verwendung

Die vorliegende Erfindung betrifft Manumycin-Derivate der allgemeinen Formel I,

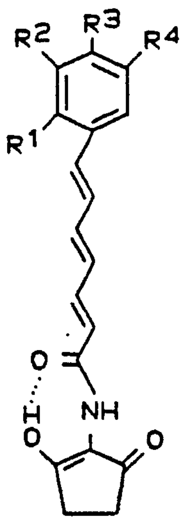

I

wobei
$R^1$ = H oder OH;
$R^2$ = H oder OH;
$R^3$ = H, OH, $OCH_3$, $CH_3$ oder $NH_2$;
$R^4$ = H, OH, $OCH_3$, $NH_2$ oder

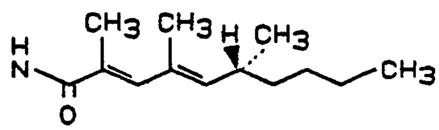

ist.

Die vorliegende Erfindung betrifft ferner ein Verfahren zur Herstellung von Verbindungen der allgemeinen Formel I, das dadurch gekennzeichnet ist, daß der Stamm Streptomyces parvulus Tü 64 (DSM 40722) in Gegenwart substituierter Benzoesäuren der allgemeinen Formel II

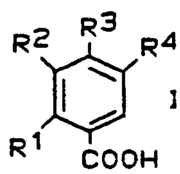

I I

wobei
$R^1$ = H oder OH;
$R^2$ = H oder OH;
$R^3$ = H, OH, $OCH_3$, $CH_3$ oder $NH_2$;
$R^4$ = H, OH, $OCH_3$ oder $NH_2$ ist,

in geeigneten Nährmedien fermentiert wird.

Die Verbindungen der allgemeinen Formel I leiten sich vom Manumycin ab (Formel III), das von F. Buzzetti et al. (Phar. Acta. Helv., 1963, 38, 871) beschrieben und dessen Struktur und absolute Konfiguration von A. Zeeck et al. (J. Antibiotics, 1987, 40, 1530 und 1549) bestimmt wurde.

III

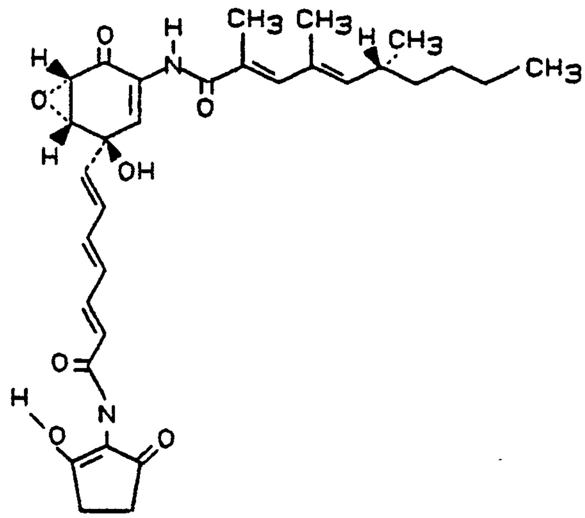

Um die normale Biosynthese der zentralen m-$C_7N$ Startereinheit für Manumycin zu umgehen wurden unphysiologische Mengen anderer $C_7$-Verläufermoleküle dem Kulturmedium während der stationären Phase des Wachstums von Streptomyces parvulus (DSM 40722) beigegeben. Das oben genannte Kulturmedium enthält neben einer Kohlenstoff- und Stickstoffquelle die üblichen anorganischen Salze. Durch Extraktion des Mycels mit einem organischen Lösemittel, das nicht oder nur wenig mit Wasser mischbar ist, erhält man farbige Extrakte, aus denen nach Abtrennen der lipophilen Bestandteile und Reinigung des Rohproduktes durch Chromatographie Verbindungen der allgmeinen Formel I erhalten werden.

Durch Hydrolyse von Verbindungen der allgemeinen Formel I mit

$R^4$ = 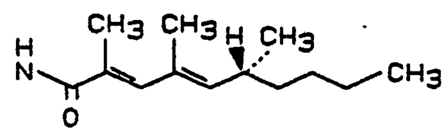

(Seitenkette des Manumycins) mit 20 %iger methanolischer KOH entstehen Verbindungen der allgemeinen Formel I mit $R^4$ = $NH_2$. Die Verbindungen (1) und (2) aus Tabelle 1 sind auch durch Reduktion des Manumycins (Formel III) nach bekannten Verfahren zugänglich, wie von A. Zeeck at al. (J. Antibiotics, 1987, 40, 1541) beschrieben. Wir haben gefunden, daß Verbindungen der allgemeinen Formel I, insbesondere die Verbindungen (1), (2), (3) und (5) in Tabelle 1 die Leukozyten-Elastase hemmen.

Tabelle 1 Verbindungen der allgemeinen Formel I mit folgenden Resten $R^1$ bis $R^4$

| | $R^1$ | $R^2$ | $R^3$ | $R^4$ |
|---|---|---|---|---|
| Verbindung (1) | OH | H | OH | 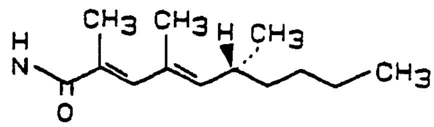 |
| Verbindung (2) | H | H | OH | 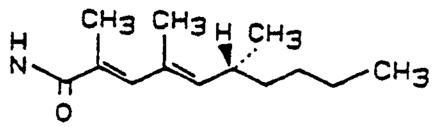 |
| Verbindung (3) (= 64-mABA) | H | H | H | 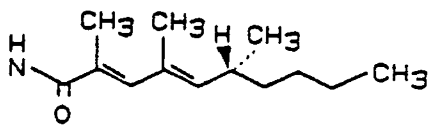 |
| Verbindung (4) | H | H | H | $NH_2$ |
| Verbindung (5) (= 64-pABA) | H | H | $NH_2$ | H |

Die zu den neutralen Proteasen zählende Leukozyten-Elastase ist im menschlichen bzw. tierischen Organismus am Abbau verschiedenster löslicher und gewebsständiger Proteine beteiligt.

Da jedoch eine unkontrollierte proteolytische Aktivität zum Abbau wichtiger Regulatorproteine (wie die des Gerinnungs-und Komplementsystems) führen würde, hält der Organismus unter physiologischen Bedingungen eine Balance zwischen dem Enzym und seinen natürlich vorkommenden Inhibitoren (wie z.B. α1-Protease -Inhibitor, α2-Makroglobulin) aufrecht. Bei verschiedenen pathologischen Zuständen kann es nun zu einer vermehrten Sekretion von Elastase aus Leukozyten (vorzugsweise aus polymorphkernigen Granulozyten) oder einer erniedrigten Freisetzung von Inhibitoren und damit zur Zerstörung von wichtigen Regulator- oder Strukturproteinen kommen.

Die Krankheitsbilder mit dieser Ätiologie sind vielfältig und können unterschiedliche Organe und Gewebe betreffen: Es gehören hierzu die verschiedenen Zustände des Schocks (z.B. septischer Schock), Störungen des Gerinnungssystems, Erkrankungen der Lunge, wie das "acute respiratory distress syndrome (ARDS)" und das Lungenemphysem, posttraumatische und postoperative Komplikationen, verschiedene Formen der Entzündung, sowohl in ihrem akuten wie in ihrem chronischen Verlauf wie die rheumatoide Arthritis und andere Collagenosen.

Des weiteren spielt die von Tumorzellen sezernierte Elastase bei der Metastasierung und dem invasiven Wachstum von Tumoren eine bedeutende Rolle, in dem sie die den Primärtumor umgebende Basalmembran zerstört und den Durchtritt von Tumorzellen durch diese Matrix erleichtert.

Entsprechend ist bei all den geschilderten Erkrankungen der Einsatz von Elastaseinhibitoren von therapeutischem Nutzen.

In den folgenden Beispielen wird die Erfindung näher erläutert.

**Beispiel 1: Herstellung von 64-mABA** (Verbindung (3), s. Tabelle 1)

a) Herstellung einer Sporensuspension des Produzentenstamms. 100 ml Nährlösung (4 g Hefeextrakt, 10 g Malzextrakt, 4 g Glucose, 1 l Leitungswasser, pH-wert vor dem Sterilisieren 7,3) in einem 500 ml Erlenmeyerkolben mit einem seitlichen Einstich werden mit dem Stamm DSM 40722 beimpft und 72 Stunden bei 28 °C und 250 UpM auf der rotierenden Schüttelmaschine inkubiert. Anschließend werden 20 ml Kulturflüssigkeit in einem 500 ml Roux-Kolben mit dem Nährboden der oben genannten Zusammensetzung, dem 20 g Agar pro Liter zur Verfestigung zugegeben wurde, gleichmäßig verteilt und dekantiert. Die

Kulturen werden 10 bis 14 Tage bei 28 °C inkubiert. Die nach dieser Zeit entstandenen Sporen eines Roux-Kolbens werden mit 500 ml entionisiertem Wasser, das einen Tropfen eines handelsüblichen nichtionischen Tensids enthält, abgeschwemmt, sofort weiterverwendet oder bei -22 °C aufbewahrt.

b) Herstellung einer Kultur bzw. Vorkultur des Produzentenstammes im Erlenmeyerkolben. Ein 500 ml Erlenmeyerkolben mit einem seitlichen Einstich und 100 ml einer Nährlösung der Zusammensetzung 2 % entfettetes Sojamehl, 2 % Mannit und Wasser ad 100 (pH 7,5 vor dem Autoklavieren) wird mit einer auf einem Schrägröhrchen gezogenen Kultur oder mit 0,2 ml Sporensuspension angeimpft und auf einer Schüttelmaschine bei 250 UpM und 28 °C inkubiert. Zum Animpfen von 10, 20, 25 und 100 l Fermentern genügt eine 48 Stunden alte Submerskultur (5 %) aus der gleichen Nährlösung.

c) Fermentation

Ein Fermenter von 15 l Fassungsvermögen wird wie folgt betrieben. Bei 28 °C Inkubationstemperatur und 250 Umdrehungen pro Minute (Medium: Sojamehl entfettet 2 %, Mannit 2 %, pH der Lösung vor dem Sterilisieren mit NaOH auf 7,5 eingestellt) werden 4 l Luft prop Minute in die Kulturflüssigkeit eingeleitet. Durch wiederholte Zugabe weniger Tropfen von ethanolischer Polyollösung kann die Schaumentwickung unterdrückt werden. Nach 30-45 Stunden, vorzugsweise 36 Stunden, werden 55 mMol 3-Aminobenzoesäure (in wenig Wasser gelöst, pH = 7) pro Liter Kulturmedium zugegeben. Nach weiteren 38 Stunden wird die Kultur mit 2 M HCl auf pH = 4,5 eingestellt, mit Hyflo-Celite (50 g pro Liter) 10 Minuten gerührt und filtriert. Der Filterrückstand wird dreimal mit Aceton (250 ml pro Liter Kulturlösung), das Kulturfiltrat dreimal mit Ethylacetat extrahiert: Die Extrakte werden eingeengt. Die wäßrig-öligen Rückstände werden vereinigt und viermal mit Chloroform (30 ml pro Liter Kulturlösung) extrahiert. Die vereinigten organischen Phasen liefern nach dem Verdampfen des Lösungsmittels ein öliges Rohprodukt, das bei der Behandlung mit eisgekühltem Petrolether fest wird. Das dunkelbraune amorphe Pulver wird zweimal über Sephadex LH-20-Säulen (100 x 2,5 cm, Auftrags- und Elutionsmittel: $CHCl_3$) chromatographiert. Daran schließt sich eine Chromatographie über eine RP-8 Silica-Gel-Säule an (Acetonitril-Wasser, 3:1 v/v) um nach einer optimalen Fermentation 123 mg/l Kulturbrühe reines, gelbes amorphes 64-mABA (Verbindung (3)) zu ergeben. Der Schmelzpunkt beträgt 196-197 °C.

Weitere Eigenschaften sind wie folgt

$[\alpha]_D^{22}$ beträgt -213° (c=0.15 in $CHCl_3$)
IR (KBr): 3420, 3260, 2940, 2910, 1605, 1575(sh) 1530 und 996 $cm^{-1}$

UV-Spektrum:
$\lambda_{max}$ (Methanol) = 351 (40 800), 263 (25 100)
$^{13}C$, $^{1}H$-NMR (200 MHz, $CDCl_3$): $\delta_c$ = 197.4 (s, C-1''), 174,3 (C-3''), 168.7 (s, C-1'), 165.9 (s, C-13), 144.0 (d, C-11), 142.1 (d, C-5'), 141.6 (d, C-9), 138.9 (d, C-3'), 138.8 (s, C-2), 137.4 (s, C-4), 136.9 (d, C-7), 130.2 (d, C-10), 130.1 (s, C-2'), 129.4 (s, C-4'), 129.3 (d, C-6), 128.5 (d, C-8), 123.0 (d, C-5), 120.4 (d, C-12), 120.1 (d, C-1), 118.0 (d, C-3), 115.3 (s, C-2''), 37.1 (t, C-7'), 32.8 (d, C-6'), 32.2 (t, C-5''), 29.8 (t, C-8'), 25.7 (t, C-4''), 22.8 (t, C-9'), 20.8 (q, C-13'), 16.6 (g, C-12'), 14.4 (q, C-10'), 14.1 (g, C-11'); $\delta_H$ ($CDCl_3$), 0.86 (t, H 6.4 Hz, 10'-$H_3$), 0.93 (d, J 6.4 Hz, 13'-$H_3$), 1.14-1.42 (m, breit, 7'-$H_2$, 8'-$H_2$, 9'-$H_2$), 1.84 (d, J 1.6 Hz, 12'-$H_3$), 2.12 (d, J 1.6 Hz, 11'-$H_3$), 2.34-2.50 (m, breit, 6'-H), 2.58-2.60 (s, breit, 4''-$H_2$, 5''-$H_2$), 5.36 (d, J 10 Hz, 5'-H), 6.09 (d, J 15 Hz, 12-H), 6.42 (dd, J 13.5 und 12 Hz, 10-H), 6.62-6.88 (m, 7-H, 9-H, einschließlich 6.82, 3'-H), 6.90 (dd, J 15.5 und 11 Hz, 8-H), 7.15 (d, breit, J 7.5 Hz, 5-H), 7.22-7.46 (m, 1-H, 11-H), 11-H), 7.64 (s, breit, NH), 7.86 (s, 3-H), 7.88 (s. breit, NH), 13.80 (s, breit, OH);

Massenspektroskopie: m/z 502
(21 %, $M^+$, gefunden: 502.2832 für $C_{31}H_{38}N_2O_4$)
417 (24 %), 324 (11 %), 193 (38 %), 123 (11 %), 109 (64 %);
Elementaranalyse: C, 74.04; H, 7.81; N, 5.49. $C_{31}H_{38}N_2O_4$ erfordert C, 74.06; H, 7.62; N, 5.57.

**Beispiel 2: Herstellung von Verbindung (4) in Tabelle 1 durch Hydrolyse von 64-mABA**

94 mg 64-mABA wurden in 50 g 20 %iger methanolischer KOH gelöst und 24 Stunden unter Rückfluß getrocknet. Die gelbe Reaktionsmischung wurde mit wäßriger Oxalsäure auf pH 2 eingestellt und zweimal mit 300 ml $CHCl_3$ extrahiert. Die vereinigten organischen Auszüge wurden mit $Na_2SO_4$ getrocknet und eingedampft, was zu einem gelben amorphen Puder führte. Dieses Rohprodukt ergab nach Säulenchromatographie über Silicagel (45 cm x 2.5 cm, $CHCCl_3$-MeOH, 9:1 v/v) drei Hauptprodukte

(a) 5.8 mg Ausgangsprodukt, RF= 0.60 ($CHCl_3$.MeOH, 9:1 v/v)
(b) 30 mg schwach gelbes Öl, RF= 0.43 ( " " " " )
(c) 34.2 mg gelbes Pulver, RF= 0.39 ( " " " " )

(b) ergab nach weiterer Reinigung an einer Sephadex LH-20 Säule (45 cm α 2.5 cm, $CHCl_3$) 20.0 mg (54 %) 2,4,6-Trimethyl-2,4-decadiensäure (Formel IV)

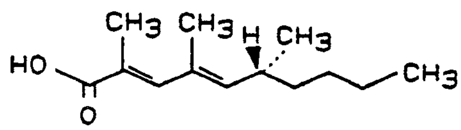
(IV)

Produkt (c) ergab nach Chromatographie über eine Sephadex LH-20 Säule (50 cm x 2.5 cm, $CHCl_3$) 302 mg (55 %) Verbindung (4) als gelbes, amorphes Pulver mit folgenden Eigenschaften:

Schmelzpunkt: 256 °C
IR (KBr): 3440, 3380, 3260, 1680 (sh) 1610, 1550 und 1008 $cm^{-1}$

Uv-spektrum in Methanol:
$\lambda_{max}$ = 342 (44 500), 259 (24 600).
$^{13}C$, $^1H$-NMR (200 MHz, DMSO-$d_6$):
$\delta_H$ 2.09 (s, 4″-$H_2$), 2.48 (s, 5″-$H_2$), 3.30 (s, breit, NH, überlappt durch HOD), 6.43-7.08 (m, 9 Protonen), 7.29 (dd, J 15.0 und 12.5 Hz, 11-H), 9.91 (s, breit, OH); $\delta_C$ 166.1 (s, C-13), 148.8 (s, C-2), 142.5 (d, C-11), 137.3 (d, C-9), 136.9 (d, C-7), 129.9 (s, C-4), 114.8 (s, C-2″), 28.8 (t, breit, C-4″, C-5″), die Signale 129.1 (d), 127.4 (d), 121.2 (d), 119.0 (d), 114.9 (d), 114.6 (d) und 112.5 (d) konnten nicht eindeutig C-1, C-3, C-5, C-6, C-8, C-10, und C-12, zugeordnet werden, die Signale für C-1″ und C-3″ wurden nicht beobachtet.

Massenspektrometrie:
m/z 310 (13 %, $M^+$, gefunden: 310.1317 für $C_{18}H_{18}N_2O_3$), 198 (49 %), 180 (15 %), 170 (92 %), 132 (100 %).

**Beispiel 3: Herstellung von 64-pABA (Verbindung (5) in Tabelle 1)**

Zu einer 36 Stunden alten Kultur von Streptomyces parvulus DSM 40722 wurden 55 mMol/Liter 4-Aminobenzoesäure gefüttert (Bedingungen wie unter 1c). Nach 74 Stunden fanden die Extraktion von Mycel und Kulturfiltrat wie oben beschrieben statt. Das dunkelbraune Rohprodukt ergab nach zweimaliger Säulen-Chromatographie über Silicagel (30 cm x 5 cm, $CHCl_3$-MeOH, 9:1 v/v) 35.8 mg/Liter 64-pABA (Verbindung (5)) als gelbes amorphes Pulver mit folgenden Eigenschaften:

Schmelzpunkt: 242 °C
IR (KBr): 3440, 3350, 3240, 2930, 2860, 1710 sh, 1615 sh, 1585, 1000 $cm^{-1}$

UV-Spektrum in Methanol:
392 (31 500), 261 (14 400), 201 (15 200)

$^{13}C$, $^1H$ NMR(200 MHz, DMSO-$d_6$): $\delta_C$ 166.4 (s, C-13), 149.6 (s, C-1), 143.1 (d, C-11), 142.5 (d, C-9), 137.8 (d, C-7), 128.3 (d, C-3 und C-5), 127.3 (d, C-10), 124.1 (s, C-4), 122.9 (d, C-8), 119.4 (d, C-12), 114.9 (s, C-2″), 113.8 (d, C-2 und C-6), 28.8 (t, breit, C-4″ und C-5″), die Signale für C-1″ und C-3″ konnten nicht beobachtet werden.

$\delta_H$ 2.10 (s, 4″-$H_2$), 2.47 (s, 5″-$H_2$), 3.30 (s, breit, NH, überlappt durch HOD), 5.50 (s, breit, NH), 6.34-6.94 (m, 7 Protonen, einschließlich 6.57 (d, J 8.5 Hz, 2-H und 6-H)), 7.23 (d, J 8.5 Hz, 3-H und 5-H), 7.30 (dd, J 14.5 und 12 Hz, 11-H), 9.94 (s, breit, OH);

Massenspektrometrie: m/z 310 (8.4 % $M^+$

Gefunden: 310.1317 für $C_{18}H_{18}N_2O_3$) 198 (10.4 %), 170 (40.7 %), 132 (100 %).

**Beispiel 4:**

Test der Elastase aus polymorphkernigen Granulozyten (PMN-Elastase)

Die Elastase wurde aus menschlichen Leukozyten nach der von Engelbrecht et al. (Hoppe-Seyler's Physiol. Chem. 363, pp. 305-315, 1982) beschriebenen Methode isoliert. Als Substrat wurde das von Nakajima et al. (J. Biol. Chem. 254, pp. 4027-4032, 1979) beschriebene MeO-Suc-Ala-Ala-Pro-Val-pNA (Calbiochem) benutzt. Die Freisetzung des p-Nitroanilins aus dem Substrat wurde als Zunahme der Absorption bei 405 nm im Spektralphotometer, innerhalb von 15 Minuten gemessen. Dieser Absorptionswert wurde als 100 % Enzymaktivität der PMN-Elastase definiert. Die Inhibitoren der PMN-Elastase wurden in steigenden Konzentrationen bis maximal 100 µg/ml für 1 Stunde mit dem Enzym vorinkubiert, die Enzymreaktion wurde mit dem Substrat gestartet. Als $IC_{50}$ wurde diejenige Inhibitor-Konzentration bezeichnet, die 50 % der Enzymaktivität inhibiert. Substanzen die bei der maximalen Konzentration von 100 µg/ml keine inhibierende Wirkung zeigten, wurden als wirkungslos definiert.

Die erhaltenen $IC_{50}$-Werte sind in der Tab. 2 zusammengefaßt. Die Referenzverbindung Manumycin zeigt vergleichbare Aktivität wie die Verbindungen (1) bis (3), während Verbindung (5) deutlich schwächer inhibierende Wirkung zeigt. Verbindung (4) ist in diesem Test unwirksam.

Tabelle 2

| Inhibierende Wirkung von Manumycin und der geprüften Manumycin-Derivate auf die PMN-Elastase | |
|---|---|
| Substanz | $IC_{50}$ (µg/ml) |
| Manumycin (Formel III) | 4.4 |
| Verbindung (1) | 2.5 |
| Verbindung (2) | 3.1 |
| Verbindung (3) (= 64-mABA) | 3.6 |
| Verbindung (4) | - |
| Verbindung (5) (= 64-pABA) | 42.0 |

## Ansprüche

1. Verbindungen der Formel I

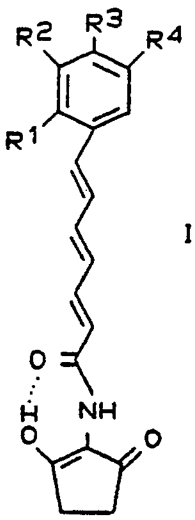

I

wobei
$R^1$ = H oder OH;
$R^2$ = H oder OH;
$R^3$ = H, OH, $OCH_3$, $CH_3$ oder $NH_2$;
$R^4$ = H, OH, $OCH_3$, $NH_2$ oder

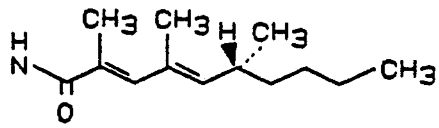

ist.

unter Ausnahme der Verbindung (1) mit $R_1$ = OH, $R_2$ = H, $R_3$ = OH und

$R^4$ = 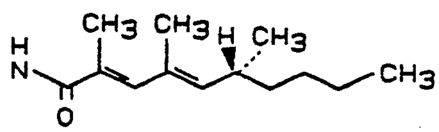

sowie der Verbindung (2) mit $R_1$ = H, $R_2$ = H, $R_3$ = OH und

$R^4$ = 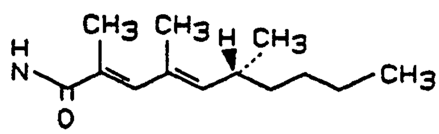

2. Verbindung der Formel I, wobei $R_1$ = $R_2$ = $R_3$ = H und

$R^4$ = 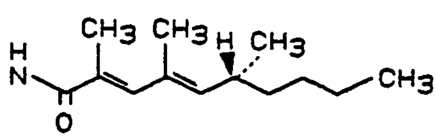

ist.

3. Verbindung der Formel I, wobei $R_1$ = $R_2$ = $R_3$ = H und $R_4$ = $NH_2$ ist.

4. Verbindung der Formel I, wobei $R_1$ = $R_2$ = $R_4$ = H und $R_3$ = $NH_2$ ist.

5. Verfahren zur Herstellung von Verbindungen der Formel I, dadurch gekennzeichnet, daß Kulturen von Streptomyces parvulus DSM 40722 jeweils substituierte Benzoesäuren der Formel II

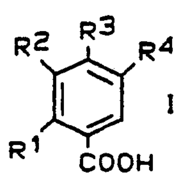

II

wobei
$R^1$ = H oder OH;
$R^2$ = H oder OH;
$R^3$ = H, OH, $OCH_3$, $CH_3$ oder $NH_2$;
$R^4$ = H, OH, $OCH_3$ oder $NH_2$ ist,

zugesetzt werden.

6. Herstellung der Verbindung des Anspruchs 2, dadurch gekennzeichnet, daß in dem Verfahren nach Anspruch 5 3-Aminobenzoesäure zugesetzt wird.

7. Herstellung der Verbindung des Anspruchs 4, dadurch gekennzeichnet, daß in dem Verfahren nach Anspruch 5 4-Aminobenzoesäure zugesetzt wird.

8. Verwendung von Verbindungen der Formel I sowie von Manumycin zur Hemmung von Elastase.

9. Verwendung von Verbindungen der Formel I als Arzneimittel.

Patentansprüche für folgenden Vertragsstaat: GR, ES

1. Verfahren zur Herstellung von Verbindungen der Formel I,

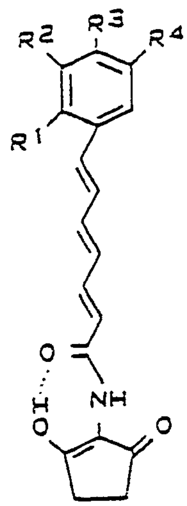

I

wobei
$R^1$ = H oder OH;
$R^2$ = H oder OH;
$R^3$ = H, OH, $OCH_3$, $CH_3$ oder $NH_2$;
$R^4$ = H, OH, $OCH_3$, $NH_2$ oder

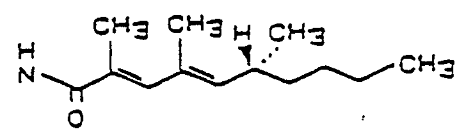

ist, dadurch gekennzeichnet, daß der Stamm Streptomyces parvulus Tü 64 (DSM 40722) in Gegenwart substituierter Benzoesäuren der allgemeinen Formel II

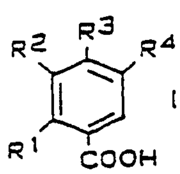

II

wobei
$R^1$ = H oder OH;
$R^2$ = H oder OH;
$R^3$ = H, OH, $OCH_3$, $CH_3$ oder $NH_2$,
$R^4$ = H, OH, $OCH_3$ oder $NH_2$ ist,

in geeigneten Nährmedien fermentiert wird.

2. Verfahren zur Herstellung der Verbindung gemäß Formel I, wobei $R_1$ = $R_2$ = $R_3$ = H und

$R^4$ = 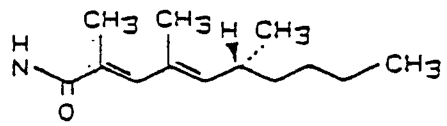

ist, dadurch gekennzeichnet, daß in dem Verfahren nach Anspruch 1 3-Aminobenzoesäure zugesetzt wird.

3. Verfahren zur Herstellung der Verbindung gemäß Formel I, wobei $R_1 = R_2 = R_4 = H$ und $R_3 = NH_2$ ist, dadurch gekennzeichnet, daß in dem Verfahren nach Anspruch 1 4-Aminobenzoesäure zugesetzt wird.

4. Verfahren zur Herstellung von Arzneimitteln, dadurch gekennzeichnet, daß Verbindungen der Formel I

wobei
$R^1$ = H oder OH;
$R^2$ = H oder OH;
$R^3$ = H, OH, $OCH_3$, $CH_3$ oder $NH_2$;
$R^4$ = H, OH, $OCH_3$, $NH_2$ oder

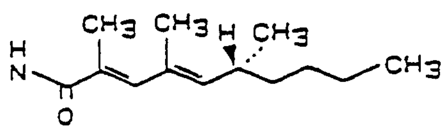

ist mit geeigneten pharmakologischen Trägern gemischt werden.